(19) Europäisches Patentamt
European Patent Office
Office européen des brevets

(11) Publication number: 0 061 761 B1

(12) **EUROPEAN PATENT SPECIFICATION**

(45) Date of publication of patent specification: **18.05.88**

(51) Int. Cl.⁴: **C 07 H 19/04** // G01N33/48, C07H21/00

(21) Application number: **82102642.4**

(22) Date of filing: **29.03.82**

(54) **A method for preparing a mononucleotide-3'-phosphodiester-based substrate.**

(30) Priority: **30.03.81 US 248688**

(43) Date of publication of application: **06.10.82 Bulletin 82/40**

(45) Publication of the grant of the patent: **18.05.88 Bulletin 88/20**

(84) Designated Contracting States: **AT BE CH DE FR GB IT LI LU NL SE**

(56) References cited:

**TETRAHEDRON LETTERS, no.33, Pergamon Press, (GB), K.K. OGILVIE et al.: "The use of silyl groups in protecting the hydroxyl functions of ribonucleosides", pages 2861-2863**

**CHEMICAL ABSTRACTS, vol.90, no.15, April 9, 1979, page 679, abstract no.121925x, Columbus, Ohio (US), K.K. OGILVIE et al.: "The synthesis of oligoribonucleotides. II. The use of silyl protecting groups in nucleoside and nucleotide chemistry, VII"**

(73) Proprietor: **Baker Instruments Corporation
2196 Avenue C. P.O. Box 2168
Bethlehem Pennsylvania 18001 (US)**

(72) Inventor: **Kang, Jemo
22 Nassau Court
Skillman New Jersey 08558 (US)**

(74) Representative: **Vossius & Partner
Siebertstrasse 4 P.O. Box 86 07 67
D-8000 München 86 (DE)**

(56) References cited:

**CHEMICAL ABSTRACTS, vol.96, no.3, January 18, 1982, page 141, abstract no.16415g, Columbus, Ohio (US), D.M. HAWLEY et al.: "Preparation properties, and uses of two fluorogenic substrates for the detection of 5'-(venom) and 3'-(spleen) nucleotide phosphodiesterases"**

**R.S. TIPSON et al.: "ADVANCES IN CARBOHYDRATE CHEMISTRY AND BIOCHEMISTRY, vol.36, 1979, Academic Press, New York (US), M. IKEHARA et al.: "The synthesis of polynucleotides", pages 135-213**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

1. Field of the Invention

The present invention relates to chromogenic and/or fluorogenic mononucleoside 3'phosphodiesters, and, more particularly, to a novel method for synthesizing such mononucleoside phosphodiesters. These materials may be used, for example, in carrying out various non-isotopic immunoassays.

2. Description of the Prior Art

For a variety of clinical purposes such as, for example, monitoring dosage schedules, monitoring hormone levels, checking for recent ingestion or following pharmacological dynamics of bioavailability, absorption, degradation or excretion, it is a great advantage to measure the concentration of various drugs to the nanomolar or even picomolar level. As is known, a radioimmunoassay can accomplish analyses of this type. To carry out an analysis, an acceptable kit or system must include an antiserum, a standard of the compound (i.e., — analyte) to be measured, the radiolabelled derivative of the compound to be measured, a buffering agent or agents and, often, a displacing agent. The antiserum is produced by bleeding animals which have been immunized by innoculation, for example, with the hapten — protein conjugate (immunogen) corresponding to the compound to be measured.

As is well known, in general, the technique of radio-immunoassay measures the competition between radioactively labelled analyte and unlabelled analyte for binding sites on the antibody in the antiserum. By adding to the antiserum known amounts of the analytes to be assayed and a radiolabelled analog, a dose-response curve for bound or free analyte versus concentration of analyte is constructed. After this immunocalibration has been carried out, unknown concentrations can then be compared to the standard dose-response curve for assay. Crucial to this type of assay is the existence of radioactive analytes which compete effectively with non-radioactive analytes. Accordingly, in order to obtain the maximum precision, accuracy, sensitivity, specificity and reproducibility of the assay, purified, well-characterized synthetic radioactive analytes are required.

Several deficiencies in radioimmunoassay methodology have been identified. First of all, it is necessary to make a physical separation of the antibody bound radiolabelled analyte from the free radiolabelled analyte. Further, the methodology is considered rather labor intensive, and the equipment required is likewise relatively expensive, is not uniformly available, and further requires the use of highly trained and skilled technicians to accurately carry out such assays. Likewise, the radioisotopically-labelled analytes are relatively unstable and expensive and pose an increasingly severe waste disposal problem owing to radiation exposure hazards associated with the commonly used radioisotopic labels. Despite these shortcomings, the use of radioimmunoassay has grown considerably.

The substantial recent growth in the use of radioimmunoassay in clinical laboratories has, however, spurred the development of variants which overcome the deficiencies of the radioimmunoassay methodology as described herein. The approaches which have been developed to overcome these deficiencies primarily involve the use of enzyme or fluorescent labels instead of radioisotopic labels, preferably coupled with conditions allowing for measuring a chemical distinction between bound and free fractions of labelled analyte which leads to the elimination of the requirement for physical separation. Immunoassays having the latter simplifying and advantageous feature are referred to as homogeneous immunoassays as opposed to heterogeneous immunoassays where physical separation is required.

Thus, homogeneous immunoassay systems have been developed which are based on the use of an enzyme-labelled analyte where the enzymatic activity of the label is decreased when complexation with the antibody occurs. Unlabelled analyte whose concentration is to be determined displaces the enzyme-labelled analyte bound to the antibody, thus causing an increase in enzymatic activity. Standard displacement or dose-response curves are constructed where increased enzymatic activity (monitored spectophotometrically using what has been termed a "substrate" which ultimately produces a unique chromophore as a consequence of enzyme action) is plotted against increased analyte concentration. These are then used for determining unkown analyte concentrations. The following United States patents have been issued in the field of homogeneous enzyme immunoassay: 3,817,837; 3,852,157; 3,875,011; 3,966,556; 3,905,871; 4,065,354; 4,043,872; 4,040,907; 4,039,385; 4,046,636; 4,067,774; 4,191,613; and 4,171,244. In these patents, the label for the analyte is described as an enzyme having a molecular weight substantially greater than 5,000. Also, commercialization of this technology has been limited so far to applications where the analytes are relatively small in molecular size at fluid concentrations of the analyte greater than $10^{-10}$M.

As a consequence of the limitations of the homogeneous enzyme immunoassay technique described above, considerable effort has been devoted towards developing more sensitive homogeneous immunoassays using fluorescence. These have been primarily directed at assays for the larger sized molecules such as immunoglobulins or polypeptide hormones such as insulin. The following United States patents have been issued for this type of assay: 3,998,943; 3,996,345; 4,174,384; 4,161,515; 4,208,479 and 4,160,016. The label in most of these patents involves an aromatic fluorescent molecule, bound either to the analyte or to the antibody. All likewise involve various methods of quenching fluorescence through antibodies or other fluorescent quenchers so that the extent of quenching is related to the amount of analyte present in the sample.

A further type of methodology which may be described as a reactant-labelled fluorescent immunoassay involves the use of a fluorescent-labelled analyte designed so that a fluorescent product is released when it is enzymatically hydrolyzed. Antibody to the analyte portion of the molecule, however, inhibits enzymatic hydrolysis. Consequently, by the law of mass action, fluorescence is enhanced in the presence of increased analyte due to enzymatic hydrolysis of the displaced, fluorescent labelled analyte. As an example, a labelled analyte is β-galactosyl-umbelliferone-sisomicin. The enzyme β-galactosidase cleaves the sugar from the umbelliferone moiety which can then fluoresce. Publications which describe this methodology include: J. F. Burd, R. C. Wong, J. E. Feeney, R. J. Carrico and R. C. Boguolaski, *Clin. Chem., 23,* 1402 (1977); Burd, Carrico, M. C. Fetter, et al., *Anal. Biochem., 77,* 56 (1977) and F. Kohen, Z. Hollander and Boguolaski, *Jour. of Steroid Biochem., 11,* 161 (1979).

EP-A-62277 provides methodology for carrying out non-isotopic immunoassays which obviates the deficiencies of prior assays of this general type. In an illustrative embodiment, this methodology utilizes a labelled analyte-polypeptide complex which expresses ribonuclease-type activity to catalytically convert a substrate to a chromogenic or fluorogenic reporter molecule.

Many organic compounds have been utilized heretofore for monitoring the catalytic activity of ribonuclease. Such organic compounds, or substrates, as they are commonly referred to, include ribonucleic acid itself, cyclic phosphate diesters, and monoribonucleotide compounds which exhibit the same or similar structural constraints as those expressed by the natural substrate.

Thus, for example, one method for monitoring the catalytic activity of ribonuclease involves the use of a ribonucleic acid solution. That method involves monitoring a decrease in absorbance at 300 nm of a ribonucleic acid solution as a function of time, M. Kunitz, *J. Biol. Chem., 164,* 563 (1946). Although that method is relatively simple to conduct, it has several deficiencies; specifically, the rate of decrease of absorption is not linear, calibration of each substate solution is required, and direct monitoring of absorbance decreases at 300 nm is impractical with clinical samples.

Another method utilized for monitoring ribonuclease activity is an end-point variant of the procedure described above. In the end point variant procedure, yeast ribonucleic acid is incubated with the enzyme sample for a fixed period of time. The remaining RNA is precipitated with perchloric acid or uranyl acetate/trifluoroacetic acid, and the absorbance of the supernatant is measured after centrifugation. S. B. Anfinsen, R. R. Redfield, W. L. Choate, A. Page, and W. R. Carroll, *Jour. Biol. Chem., 207,* 201 (1954). However, that method is much too cumbersome for homogeneous immunoassays of the type described in EP-B-62277 primarily due to the precipitation step involved.

Yet another variation of the above procedures has been reported by R. C. Kamm, A. G. Smith, and H. Lyons, *Analyt. Biochem., 37,* 333 (1970). The method described therein is based on the formation of a fluorescent reaction product resulting from the reaction of the dye ethidium bromide with intact yeast ribonucleic acid, but not with the hydrolysis products. In that method, a fluorescent signal, which is monitored, decreases with time. However, monitoring a fluorescent signal which decreases with time is disadvantageous, as the method may result in a lack of sensitivity when only modest differences in enzyme concentration are encountered. In addition, other disasdvantages are that the rate of decrease of absorption is not linear, and calibration of each substrate solution is required.

Another known substrate for monitoring ribonuclease A activity is a mononucleotide substrate, cytidine 2',3'-phosphate diester, E. M. Crook, A. P. Mathias, and B. R. Rabin, *Biochem. J., 74,* 234 (1960). In that method, an increase of absorbance at 286 nm, corresponding to the hydrolysis of the cyclic phosphate ring, is monitored over a two-hour period to measure the ribonuclease activity of the sample. This method, however, cannot be used in homogeneous immunoassay methods of the type described in the above-mentioned EP-A-62277 because there are analyte sample interferences which occur at 286 nm. Furthermore, the distinction between the substrate and product absorbance spectra is small, with the ratio of extinction coefficients being only 1.495 at 286 nm.

Further, certain mononucleoside-3'-phosphodiesters, including, 1-naphthyl esters of 3'-uridylic, 3'-inosonic and 3'-adenylic acids have been utilized as ribonuclease substrates. These napthyl esters have been used to differentiate substrate specificities of ribonucleases from various sources. H. Sierakowska, M. Zan-Kowalczewska, and D. Shugar, *Biochem. Biophys. Res. Comm., 19,* 138 (1965); M. Zan-Kowalczewska, A. Sierakowska, and D. Shugar, *Acta. Biochem. Polon., 13,* 237 (1966); H. Sierakowska and D. Shugar, *Acta. Biochem. Polon., 18,* 143 (1971); H. Sierakowska, H. Szemplinska, D. Shugar, *Biochem. Biophys. Res. Comm. 11,* 70 (1963). As a result of ribonuclease-induced hydrolysis, the use of such substances results in the liberation of 1-naphthol which is allowed to react with a diazonium salt to form an azo compound having strong visible absorbance. This approach requires that the assay kit include a separately packaged dye former (viz. — a diazonium salt). Also, this substrate cannot be employed in a fluorometric mode.

Various syntheses have been developed heretofore for the preparation of mononucleoside-3'-phosphodiesters. One such method for the preparation of uridine-3'-(1-naphthyl)phosphate is that disclosed in R. Kole and H. Sierakowska, *Acta Biochim. Polon, 18,* 187 (1971). In accordance with the method shown therein, uridine is acetylated at the 3'-hydroxyl position:

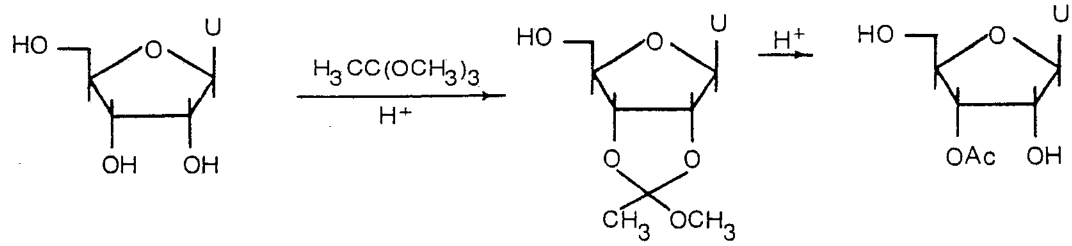


Next the 2′- and 5′-hydroxyl groups of 3′-O-acetyl uridine are blocked with dihydropyran; and sequentially, the 3′-O-acetyl undergoes hydrolysis so that 2′,5′-bis-O-(tetrahydropyranyl)uridine is formed:

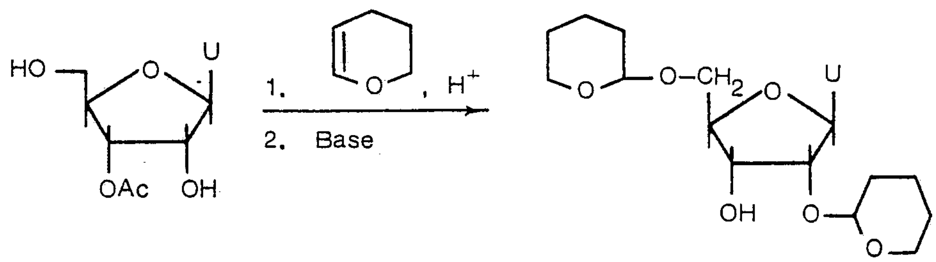


Condensation of 2′,5′-bis-O-(tetrahydropyranyl)uridine with naphthyl phosphate/dicyclohexylcarbodiimide or naphthyl phosphoryldichloride then results in 1-naphthyl phosphorylation of the 3′-hydroxyl to form the blocked form of the substrate 2′,5′-di-O-(tetrahydropyranyl)uridine-3′-(1-naphthyl)phosphate:

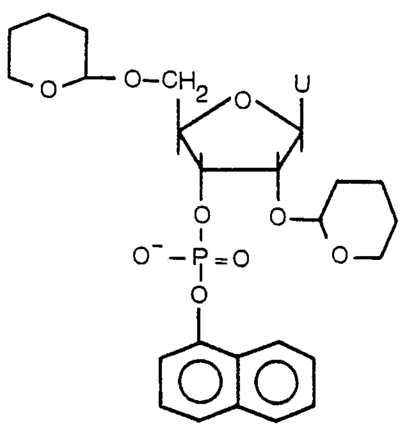

The tetrahydropyranyl blocking groups are acid labile and may be removed without competitive phosphate hydrolysis to form the substrate, uridine-3′-(1-naphthyl)phosphate:

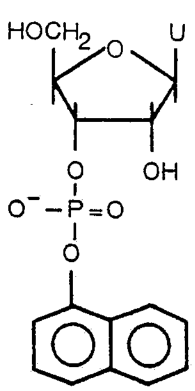

A variation of the synthesis described in Sierakowska and Shugar discussed above, is the method described in Rubsamen, Khandler and Witzel (Hoppe-Seyler's) *Z. Physiol. Chem., 355*, 687 (1974). There, 2′,5′-bis-O-(tetrahydropyranyl)-3′-uridine phosphate is prepared by the reaction of dihydropyran with uridine-3′-phosphate. Dephosphorylation of the 2′,5′-bis-O-(tetrahydropyranyl)-3′-uridine phosphate with,

for example, phosphatase or lead (II) hydroxide, forms 2',5'-di-O-(tetrahydropyranyl)uridine. The 3'-hydroxyl of that compound may then be phosphorylated in the fashion disclosed in Sierakowska and Shugar to form the desired mononucleoside-3'-phosphodiester, such as, for example, uridine-3'-(1-naphthyl)phosphate.

The synthesis schemes described by Sierakowska et al., and Rubsamen et al., suffer, however, from several major deficiencies. For example, in each synthesis method, the preparation of the key intermediate, 2',5'-bis-O-(tetrahydropyranyl)uridine, involves an undesirable, lengthy chromatography. Further, the resulting product is a mixture of diastereomeric pairs in low yields; and this complicates subsequent synthetic steps. Finally, the overall synthesis is labor-intensive.

Closely similar schemes to those of Sierakowska et al. and Rubsamen et al. are disclosed in Polish Patent No. 81969. In one synthesis described therein, uridine 2',5'-di-O-tetrahydropyrano-3'-(1-naphthyl)-phosphate is formed in dicyclohexylcarbodiimide and pyridine by the reaction of a salt of 1-naphthylphosphoric acid, (e.g., the pyridine, aniline, lutidine or tri-n-butylamine salt of the acid) with 2',5'-di-O-(tetrahydropyranyl)uridine. In another synthesis described therein, uridine 2'-O-tetrahydropyranyl-5'-O-methyl-3'-(1-naphthyl)phosphate is formed in pyridine by the reaction of a salt of 1-naphthylphosphoric acid and 5'-O-methyl-2'-O-(tetrahydropyranyl)uridine. These schemes likewise suffer from the deficiencies of the Sierakowska et al. and Rubsamen et al. methods.

In addition, methods are known for preparing oligoribonucleotides which incorporate the synthesis of 2',5'-diblocked nucleotides as intermediates. Thus, in J. Smrt and F. Sorm, *Collection Czechoslov. Chem. Commun. 27*, 73 (1962), uridylic acid is converted into 5'-O-acetyluridine 2',3'-cyclic phosphate which, after enzymatic cleavage of the cyclic phosphate by pancreatic ribonuclease, results in 5'-O-acetyluridine 3'-phosphate, which is then transformed into 2'-O-tetrahydropyranyl-5'-O-acetyluridine 3'-phosphate by the reaction with dihydropyran.

In this method, acetylation at the 5'-hydroxyl of the cyclic phosphate is utilized as a synthetic convenience for preparing intermediates in the synthesis of oligoribonucleotides. Deblocking of the 5'-acetyl is ultimately carried out in the formation of the desired oligoribonucleotide. This, however, does not describe a suitable method for synthesizing a chromogenic and/or fluorogenic mononucleoside-3'-phosphodiester.

In Tetrahedron Letters 1974, no. 33, p. 2861-2863 the use of tert.-butyl dimethylsilyl and triisopropylsilyl groups for the protection of 2'- and 2',5'-positions in ribonucleosides is described.

Further, in K. K. Ogilvie, S. L. Beaucage, A. L. Schifman, N. Y. Theriault and K. L. Sadana, *Can. J. Chem., 56* 2768 (1978), 2',5'-di-*t*-butyldimethylsilyl blocked uridine and adenosine nucleosides are prepared by the reaction of *t*-butyldimethylsilyl chloride in pyridine or imidazole with a uridine or adenosine nucleoside. The resulting silylated nucleosides are then coupled to one another by phosphorylation to form oligonucleotides.

Moreover, insofar as is known, the Smrt et al. and Ogilivie et al. methods have not heretofore been utilized in preparing such chromogenic and/or fluorogenic mononucleoside 3'-phosphodiesters, despite the deficiencies of prior methods.

Thus, despite the considerable number of methods that have been developed and utilized for synthesizing various substrates suitable for use for monitoring enzymatic or catalytic activity, there remains the need for further development which can overcome the various shortcomings of the presently known synthetic methods. None of the synthesis schemes described heretofore are currently being used commercially for the manufacture of mononucleotide-3'-phosphodiesters insofar as is known.

It is, accordingly, an object of the present invention to provide a novel method for synthesizing mononucleoside-3'-phosphodiesters having a chromogenic and/or fluorogenic functional group at the 3' phosphate moiety in the furanoside ring as disclosed in EP—A—61762 in a more direct manner involving fewer synthetic steps than required in prior methods.

Another object of this invention is to provide a novel method for synthesizing chromogenic and/or fluorogenic mononucleoside 3'-phosphodiesters for use in monitoring catalytic or enzymatic activity, which is less labor intensive than previous syntheses heretofore known.

Yet another object of this invention is to provide a novel synthesis of chromogenic and/or fluorogenic mononucleoside 3'-phosphodiesters which results in improved overall yields of desired substrate.

Still another object is to provide a novel synthesis of chromogenic and/or fluorogenic mononucleoside-3'-phosphodiesters, which may be carried out on a multigram scale sufficient for commercial use.

A further object of the present invention is to provide a product capable of being stored for extended periods of time and then, when needed, may be converted to an active form for use.

These and other objects and advantages of the present invention will become apparent from the following detailed description.

While the present invention will be primarily described in conjunction with the formation of a uridine-3'-phosphodiester, it should be appreciated that bases other than uracil may be employed, as will be described herein.

## Summary of the Invention

In general, the present invention is predicated on the discovery that mononucleoside-3'-phosphodiester substrates having a chromogenic and/or fluorogenic functional group at the 3′-phosphate moiety may be readily synthesized in as few as three steps by simultaneously blocking the 2′-, 5′-hydroxyls of a mononucleoside with a selected common blocking group and thereafter converting the diblocked species to incorporate the desired chromogenic and/or fluorogenic moiety at the 3′-position. The resulting product is provided in a form capable of being stored for extended periods of time without adverse affects and may then be converted to a useful form by removing the blocking group at least at the 2′-position. The necessary synthetic steps may be accomplished in a variety of schemes, providing sufficient versatility to allow a synthesis tailored to the desired diester substrate.

The resulting chromogenic and/or fluorogenic mononucleotide-3′-phosphodiester substrates are capable of undergoing enzymatic hydrolysis of the phosphate ester at the 3′-position to cleave a chromophore/fluorophore phosphate linkage to yield a species capable of being directly monitored spectrophotometrically or fluorometrically. They may be utilized for monitoring the catalytic activity of ribonuclease A, and/or polypeptide having the catalytic activity of such enzyme. The chromogenic and/or fluorogenic mononucleotide substrates formed by the method of this invention are useful in the immunoassay methodology disclosed in the previously identified EP—A—62277.

Detailed Description of the Preferred Embodiments

In accordance with one embodiment of the present invention, suitable starting materials comprise mononucleosides having the following structural formula:

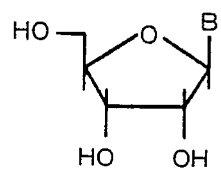

In this structure, there appear to be certain steric constraints which must be met in order to ultimately provide a substrate suitable for monitoring the catalytic activity of, for example, ribonuclease A-induced hydrolysis. Thus, the *trans, cis* orientation of the base B and substituents at positions 1′- and 2′-, 3′-, respectively, appear to have rigid structural constraints to provide a suitable substrate. However, the substituents at the 4′-position, that is, $CH_2OH$, may apparently have a configuration where the $CH_2OH$ group is *cis* to both the 2′- and 3′-functional groups, without affecting the desirable attributes of the substrate. A. Holy and F. Sorn, *Biochemica. Biophysica. Acta., 161*, 26 (1968). Accordingly, while the method of the present invention will be described in conjunction with the preparation of a substrate wherein the 4′-$CH_2OH$ substituent is *trans* to the 2′, 3′-substituents, it should be appreciated that the method is likewise equally applicable to the preparation of a substrate wherein the 4′-$CH_2OH$ substituent is *cis* to the 2′-, 3′-substituents.

From the functional standpoint, the selection of the base should take into account the following factors, in addition to, of course, its effect on product stability: (1) any modulation (increase or decrease) of catalytic activity, (2) the difficulty of synthesis, (3) the effect on endogenous enzymatic activity and (4) the solubility in aqueous or other mediums of interest should not be adversely affected to any significant extent. Other factors to consider include possible effects on hydrolysis and non-specific medium induced hydrolysis.

A wide variety of pyrimidine analogs are useful bases, including uracil, dihydrouracil, cytosine, dihydrocytosine and halogenated uracils. Additionally, based on data extrapolated from results on the ribonuclease-induced hydrolysis of both the natural substrate, RNA, as well as various synthetic substrates, such as, for example, nucleotide homopolymers, F. M. Richards and W. W. Wykoff in *The Enzymes*, (P. D. Boyer, Ed.), Academic Press, 3d Edition, Volume 4, pages 647—806, London and New York (1978), the following pyrimidine analogs should be suitable bases:

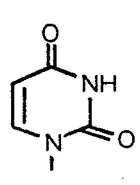
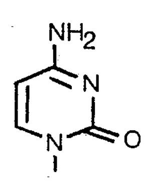
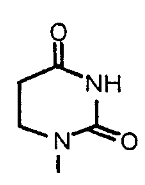
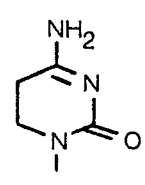
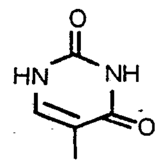
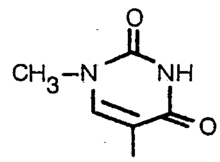
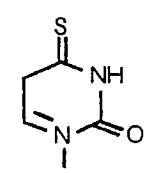
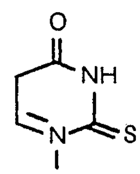

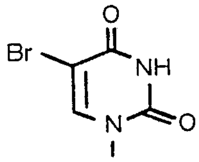
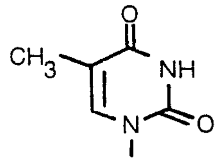
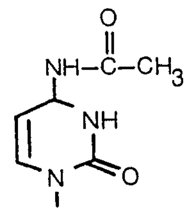

The use of purine analogs as bases, such as, adenosine and guanosine, does not provide active substrates for monitoring the catalytic activity of ribonuclease A. Further, any other pyrimidine analogs may be used consistent with the functional considerations set forth herein.

In carrying out the first step of the method, the mononucleoside is reacted with a silylating reagent to form a 2′-, 5′-disilylblocked mononucleoside of the general formula:

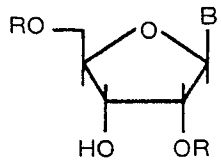

Suitable blocking groups R should meet the following criteria: (1) readily introduced without affecting the other key functionalities, (2) compatible with the subsequent phosphodiester formation step, and more particularly, should minimize or eliminate undesired side reactions in such step, (3) sufficiently stable to allow long-term storage without any adverse deleterious affects, (4) easily removed without disruption of the phosphodiester bond and (5) being capable of at least blocking medium-induced hydrolysis of the phosphate ester at the 3′-position, and the blocking group at least at the 2′-hydroxyl being capable of being removed to provide a substrate characterized by the ability to undergo enzymatic hydrolysis of the phosphate at the 3′-position. These criteria are satisfied by various silyl derivatives including triisopropylsilyl, *tert*-butyltetramethylenesilyl and *tert*-butyldimethyl silyl. The *tert*-butyldimethyl silyl moiety is preferred.

In carrying out the silylation step, the resulting products comprise, in addition to the desired 2′-, 5′-diblocked species, a series of isomers including the 3′, 5′-; 2′-, 3′-, 5′- and 2′-, 3′-diblocked species. The principal isomers provided will typically be the desired 2′-, 5′-diblocked species and the 3′-, 5′-diblocked product. The product mixture obtained will depend upon the particular process parameters employed, and selectivity to the desired species may be enhanced by appropriate selection of such process parameters. Regardless, the separation of the various isomers to provide the desired diblocked species is relatively simple (e.g. — carried out by straightforward known chromatographic methods) and is not unduly complicated even where selectivity is considerably less than optimum, as might occur when the reaction time is relatively short.

As one illustrative example, the silylation step may be carried out by using a relative molar ratio of mononucleoside to silylation reagent of 1:3 to 1:4 in pyridine, which serves both as a solvent and as a base for catalyzing the reaction. The reaction proceeds satisfactorily at ambient temperatures and will provide high selectivity and conversion for the desired species after a reaction time of 60 hours or so.

The particular process parameters can be varied within wide limits, as may be desired. A variety of solvents are useful, including dimethylformamide, dimethylsulfoxide, tetrahydrofuran, dioxane and the like. Other useful catalysts include imidazole, 2,6-lutidine, triethylamine or the like. Useful temperatures range from 10°C. to 50°C. with reaction times ranging from no more than 20 minutes or so to as much as 100 hours or so.

The second step of the procedure in accordance with the present invention involves the formation of 2′-, 5′-diblocked chromogenic and/or fluorogenic mononucleoside-3′-phosphodiester by the reaction of the 2′-, 5′-diblocked mononucleoside with a suitable derivative form of the chromophore and/or fluorophore, as depicted below:

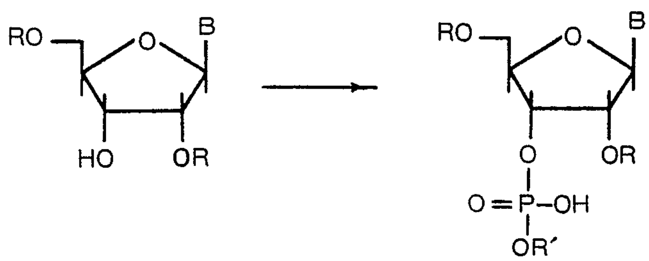

The mononucleoside-3′-phosphodiesters so formed may be readily handled and chromatographed due to their enhanced solubility in organic solvents, if desired.

The R′ group may be umbelliferonyl, 4-methylumbelliferonyl, 3-flavonyl, ortho, meta or para nitrophenyl, dinitrophenyl, cyanophenyl, acylphenyl, carboxyphenyl, phenylsulfonate, phenylsulfonyl or phenylsulfoxide.

The formation of the desired phosphodiester should be conducted, of course, in a fashion adequate to insure that no or only a minimum disruption of the key functionalities occurs. Desirably, the formation should likewise allow use of relatively mild reaction conditions to provide a product capable of being readily isolated in high yields. These general objectives may be suitably accomplished by, in general, either reacting the 2′,5′-diblocked mononucleoside directly with a phosphorylated derivative of the desired chromophore and/or fluorophore or by first converting the 2′,5′-diblocked mononucleoside to a 3′-phosphorylated derivative and then reaction with an alcohol derivative of the chromophore and/or fluorophore.

The first approach can be carried out by two synthetic schemes. One scheme involves reaction of the phosphate derivative of the chromophore/fluorophore with the 2′-, 5′-diblocked mononucleoside in the presence of a suitable condensation agent. Importantly, the reagent selected should provide a high yield of ester product at mild reaction conditions. Toluenesulfonyl chloride, mesitylenesulfonyl imidazolide, p-toluenesulfonyl imidazolide, 2,4,6-triisopropylbenzenesulfonyl chloride, mesitylenesulfonyl chloride, picrylsulfonyl chloride, N,N′-dicyclohexylcarbodiimide, 1-(3-dimethylaminopropyl)-3-ethylcarbodiimide hydrochloride, and other carbodiimide analogs with or without additives such as, N-hydroxy-succinimide, and N-hydroxyphthalimide are illustrative examples. The use of 2,4,6-triisopropylbenzenesulfonyl chloride and N,N′-dicyclohexylcarbodiimide have been found to be satisfactory.

The relative ratio of 2′-, 5′-diblocked mononucleoside to the phosphorylated chromogenic and/or fluorogenic derivative should be in the molar range of at least 1 to 1, desirably employing an excess of such derivative. A ratio of 1:2 has been found satisfactory for the amount of the nucleoside to that of the condensation agent, but excesses up to perhaps 1:5 or so may likewise be perhaps useful.

The reaction may be carried out in an aprotic polar solvent such as N,N-dimethylformamide, dioxane or tetrahydrofuran and the like, in the presence of a base such as pyridine, triethylamine and the like. Suitably, dry pyridine base as a solvent may be used at a temperature in the range of from about −20°C. to about 25°C. with a reaction time of 5 to 18 hours. A reaction temperature range of from about −20°C. to about 50°C. and a time period of about 2 to 72 hours should likewise be satisfactory.

The second synthetic scheme involves starting with a chromogenic and/or fluorogenic alcohol. The alcohol may first be phosphorylated *in situ* to form a reactive intermediate which is then reacted with the 2′-, 5′-disilylated blocked mononucleoside to form the phosphodiester. The phosphorylation reaction may be carried out by employing any of the numerous phosphorylation reagents known in the art, such as, for example, phosphorous oxychloride, 2,2,2-trichloroethyl phosphorodichloridite, or the like. The phosphorylation is carried out, typically utilizing an excess of the phosphorylation reagent, in an aprotic solvent such as N,N-dimethylformamide, dioxane, tetrahydrofuran or the like, in the presence of a base, such as, pyridine or triethylamine. Pyridine may be used as the solvent and base. The excess phosphorylating reagent may then be removed from the reactive intermediate before further reaction to form the 2′,5′-disilylblocked mononucleoside phosphodiester.

Suitable reaction conditions include employing dry pyridine base at a temperature in the range of from −10°C to 30°C and a reaction time of 1 to 3 hours. A temperature range of −78°C to 80°C and reaction times of from about 5 minutes to about 5 hours should likewise be suitable.

In a first alternative, but related aspect of the invention, the 2′,5′-disilylated blocked mononucleoside may be phosphorylated to form a diblocked mononucleotide intermediate, which may then be reacted with a chromogenic and/or fluorogenic alcohol to form the 2′,5′-disilylated blocked mononucleoside phosphodiester.

In the second approach, the 2′,5′-diblocked nucleoside derivative is first phosphorylated to form a reactive intermediate which is then reacted with the chromophore and/or fluorophore alcohol. The phosphorylation and subsequent ester forming reactions may suitably be carried out as previously described in conjunction with the first approach. The phosphorylated nucleoside derivative can be either the acid chloride or the acid itself. In the latter instance, a condensation reagent should be employed, as has been previously described in conjunction with the first approach.

In accordance with yet another embodiment of the present invention, a 2′,5′-disilylblocked mononucleotide is first prepared by the reaction of a 3′-mononucleotide and a silylating reagent, as shown schematically below:

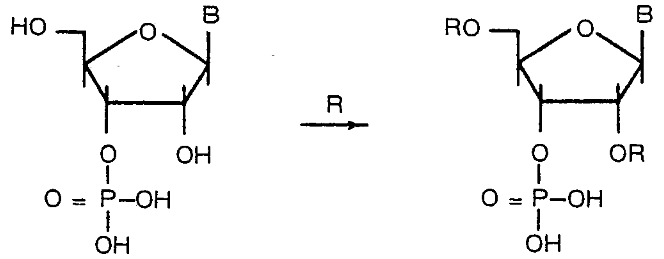

wherein R is a silyl blocking group, and B is a base, as previously defined herein. The 2′,5′-disilylblocked mononucleotide may then be condensed with the chromogenic and/or fluorogenic alcohol to form the 2′,5′-disilylblocked mononucleoside 3′-phosphodiester. The silyation and condensation reactions may be carried out utilizing the process parameters previously discussed in the first approach.

The 2′,5′-disilylblocked substrate is a stable compound which may be stored for extended periods of time. However, deblocking is necessary to provide a suitable substrate for use in monitoring the enzymatic or catalytic activity in applications such as non-isotopic immunoassays.

Deblocking of the substrate may be readily carried out with the use of several different reagents without significant deleterious hydrolytic cleavage of the deblocked substrate.

The reagents should, of course, be mild enough so that the phosphate diester bond is not cleaved during the deblocking step. Also, and importantly, the reagent should be capable of being readily separated from the phosphodiester substrate and should not inhibit or otherwise interfere with the subsequent intended application. Suitable reagents include acids, ammonium halide salts, and inorganic halide salts. Generally, *tetra*butylammonium fluoride, tritylfluoroborate, lithium tetrafluoroborate, hydrogen fluoride, acetic acid, and hydrochloric acid may be used. *Tetra*butylammonium fluoride has been found suitable.

The deblocking reaction is generally carried out in a protic or aprotic polar solvent such as tetrahydrofuran, acetonitrile, dioxane, pyridine or water using an excess of the deblocking reagent. As an example, a 1 M solution of tetrabutylammonium fluoride in tetrahydrofuran may be employed at a temperature of 15°C. to 30°C. for a period of from 20 minutes to 50 minutes. The temperature range may suitably extend from 0°C. to 50°C. with reaction times of as little as 10 minutes up to perhaps 120 minutes or so.

The following Examples are merely illustrative of the present invention and are not intended as a limitation on the scope thereof.

### Example I

This Example illustrates the preparation of 2′,5′-bis-*t*-butyldimethylsilyluridine.

In the preparation of 2′,5′-*bis*-*t*-butyldimethylsilyl-uridine, 11.39 g, 0.0466 mole, of uridine was dissolved in 80 ml of pyridine by stirring at room temperature for about 5 min. Then 21.09 g, 0.140 mole, *t*-butyldimethylsilyl chloride was added to the pyridine solution and the mixture was stirred at room temperature for about 62 hours in a flask fitted with a drying tube. The reaction mixture was diluted with 150 ml ether and then filtered to remove pyridine-HCl. The ether-pyridine filtrate was concentrated on a rotary evaporator and then in high vacuum using a liquid nitrogen trap.

Thin layer chromatography of an aliquot of the reaction product mixture on silica gel plate, with a solvent of, by volume, two parts ether and one part hexane showed three components, respectively, at $R_f$ 0.65, 0.5 and 0.3.

The remainder of the oily reaction product mixture was chromatographed on a 4.2 × 44 cm silica gel column comprising Silica gel 60 (EM Reagent, Lot No. 7953179), of particle size 0.063—0.2 mm and 70—230 mesh (ASTM) with a solvent of, by volume, two parts hexane and one part ethyl acetate, to separate the three components of the reaction product mixture. The fractions having $R_f$ of 0.5, identified by thin layer chromatography at the conditions given above, were combined. Additionally, fractions containing the $R_f$ 0.3 and 0.65 components were rechromatographed to isolate additional $R_f$ 0.5 product. The $R_f$ 0.5 fractions were combined to give a yield of 8.961 g, that is 40.5%. The melting point (123—125°C.) and n.m.r. spectrum ($CDCl_3$) of the product confirmed the product as 2′,5′-*bis*-*t*-butylmethylsilyluridine.

### Example II

This Example illustrates the preparation of 2′,5′-bis-*t*-butyldimethylsilyl-3′-uridine 1-naphthyl phosphate.

The disodium salt of 1-naphthyl phosphate, 1.814 g, 6.77 mmoles, was converted into the pyridinium salt using a Bio-Rad AG$^R$ 50 W—X8 cation exchange column. The resulting pyridinium salt solution was concentrated at room temperature in vacuum.

A solution of 3.197 g, 6.77 mmoles of 2′,5′-*bis*-*t*-butyldimethylsilyluridine, prepared in Example I, in 50 ml of dry pyridine was added to a solution of the concentrated pyridinium salt in 50 ml dry pyridine. The mixture was dried twice by stripping pyridine off, using 50 ml of dry pyridine for each drying operation. The resulting glassy residue was redissolved in 10 ml of dry pyridine and 2.6 g of 2,4,6-triisopropylbenzenesulfonyl chloride was added to the solution. The reaction mixture was stirred in the dark at room temperature for 20 hours and then concentrated to dryness *in vacuo* at room temperature.

Thin layer chromatography of a portion of the crude product on silica gel using a solvent system comprised of hexane, methanol, methylene chloride and triethylamine in the ratio, by volume, of 5:2:2:0.5, showed one major spot at $R_f$ 0.5 and minor spots at the origin, $R_f$ 0.81 and $R_f$ 0.75. Under these conditions, the starting material had an $R_f$ of 0.75, and the diester had an $R_f$ of 0.5.

The crude mixture was then chromatographed on a 23 × 2.5 cm Silica Gel G column to separate the components. The column was eluted sequentially with 100 ml of chloroform, 100 ml of 5% methanol in chloroform, and 80% methanol in chloroform until the eluant showed product. Eluant fractions of twenty ml each were collected. Fractions 10 to 18 were identified by thin layer chromatography at the conditions given above, as containing the phosphodiester product ($R_f$ 0.5). These fractions were combined and

concentrated to give 4.721 g of tan colored product. The melting point, 83—86°C., and infrared spectrum (in KBr) and n.m.r. spectrum ($CDCl_3$) of the product confirmed the product as 2′,5′-*bis-t*-butyldimethylsilyl-3′-uridine 1-naphthyl phosphate.

Example III

This Example illustrates the preparation of 3′-uridine-(1-naphthyl)phosphate.

The 2′,5′-*bis-t*-butyldimethylsilyl-3′-uridine-(1-naphthyl)phosphate prepared in Example II, 75 mg, was treated with 3.2 ml of 1 M solution of tetrabutylammonium fluoride in tetrahydrofuran. The mixture was stirred at room temperature for 40 minutes, and then the solvent was removed by evaporation *in vacuo* to leave the crude product residue. The residue was dissolved in 2 ml of water, and extracted 3 times with 5 ml portions each of ether to remove unwanted byproducts. The aqueous solution was blown by a stream of nitrogen to remove any residual ether to thus obtain 2′-uridine-(1-naphthyl)phosphate solution, essentially free of byproducts.

The 3′-uridine 1-naphthyl phosphate solution was buffered with 0.1 M sodium acetate buffer of about pH 5 and used in the thyroxine assays set forth in Examples XVII, XVIII and XXIX in the previously identified copending Farina et al. application.

Example IV

This Example illustrates the preparation of 2′,5′-*bis-tert*-butyldimethylsilyl-3′-uridine (4-methylumbelliferone-7-yl)phosphate.

In this Example, 2′,5′-*bis-tert*-butyldimethylsilyluridine is phosphorylated to form a reactive intermediate which is reacted with 4-methylumbelliferone.

In a round bottom flask, 0.2386 g of 2′,5′-*bis-tert*-butyldimethylsilyluridine, prepared in Example I, was dissolved in 5 ml of dry pyridine. The solution was evaporated to dryness *in vacuo*. The solid residue was redissolved in 7 ml of dry tetrahydrofuran and 4 ml of pyridine, and cooled with stirring in an ice-water bath under exclusion of atmospheric moisture. To the stirred cold solution there was added 0.5 ml of phosphorus oxychloride, using an air tight syringe. The mixture was allowed to stir for 5 minutes in a cooling bath, and then at room temperature for 1.5 hours. Pyridine HCl salt was deposited in the bottom of the flask.

An aliquot of the reaction mixture was analyzed by thin layer chromatography to monitor the formation of the intermediate. The chromatography was carried out on a silica gel plate with a solvent system comprising ethyl acetate, chloroform and hexane in the ratio, by volume, of 5:2:3. The analysis showed a component with $R_f$ near the origin. However, there was no component with $R_f$ 0.55 thereby indicating that the uridine starting material had been completely consumed.

The remainder of the reaction mixture was concentrated *in vacuo* using a liquid nitrogen trap to remove unreacted phosphorus oxychloride. To the residue there was added 0.107 g of 4-methylumbelliferone, and the mixture was cooled in an ice-water bath under nitrogen atmosphere to exclude atmospheric moisture. To the mixture were was added 4 ml of dry pyridine, and the resulting solution was stirred at room temperature for 40 minutes.

An aliquot of the resulting light yellow solution was analyzed by thin layer chromatography, at the same conditions as given above. A new fluorescent spot, believed to be 2′,5′-*bis-tert*-butyldimethylsilyl 3′-uridine(4-methylumbelliferone-7-yl)phosphate, was found.

The remainder of the solution was concentrated *in vacuo* to a glassy oil. The oil was suspended in 5 ml of tetrahydrofuran (THF). To the THF suspension, there was added 20 ml of ether and the mixture was stored in a cold room, about 4 to 8°C., to precipitate product. The product as obtained in this fashion was collected by filtration and dried over $P_2O_5$ *in vacuo* to yield 0.572 g of light gray powder. The product was confirmed by n.m.r. to contain 2′, 5′-*bis-tert*-butyldimethylsilyluridine 3′-(4-methylumbelliferone-7-yl) phosphate.

The 2′, 5′-*bis-tert*-butyldimethylsilyl-uridine-3′-(4-methylumbelliferone-7-yl) phosphate was deblocked following the same procedure as set forth in Example III, to form 3′-uridine-(4-methylumbelliferone-7-yl) phosphate, which was identified by enzyme assay. In an assay with RNase enzyme, the assay mixture was excited at 325 nm and emmision was monitored at 450 nm corresponding to the fluorogenic 4-methylumbelliferone, resulting from catalytic hydrolysis of 3′-uridine-(4-methylumbelliferone-7-yl) phosphate.

**Claims**

1. A method for preparing a mononucleoside-3'-phosphodiester-based substrate comprising
(a) blocking both the 2'- and 5'-hydroxyl groups of a mononucleoside of the formula:

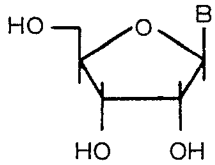

wherein B is a pyrimidine analogue, and wherein the $CH_2OH$ group at the 4'-position is either *cis* or *trans* to said base and the hydroxyl groups at the 2' and 3'-positions are *trans* to the base, by reacting said mononucleoside with a silyating reagent blocking member to form a 2',5'-diblocked mononucleoside of the formula:

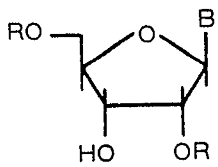

wherein R is a silyl blocking member; and
(b) forming a 2',5'-diblocked mononucleoside 3'-phosphodiester of the formula

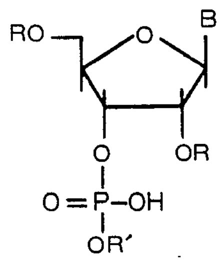

wherein R' is a chromophore or fluorophore moiety selected from umbelliferonyl, 4-methylumbelliferonyl, 3-flavonyl, o-nitrophenyl, m-nitrophenyl, p-nitrophenyl, 2,4-dinitrophenyl, cyanophenyl, acylphenyl, carboxyphenyl, phenylsulfonate, phenylsulfonyl and phenylsulfoxide, by a process selected from

1) reacting the 2',5'-diblocked mononucleoside with a phosphate derivative of one of the above chromophore or fluorophore moieties in the presence of a condensation agent to form the desired phosphodiester, or

2) phosphorylating the 2',5'-diblocked mononucleoside and reacting the phosphorylated 2',5'-diblocked mononucleoside with one of the above chromophores of fluorophores in alcoholic form to form the desired phosphodiester.

2. The method of claim 1 comprising the further step of reacting said 2',5'-diblocked mononucleoside 3'-phosphodiester with a deblocking agent to remove the silyl blocking member at least at the 2'-hydroxyl.

3. The method of claim 1 wherein said pyrimidine analogue is a member selected from uracil, dihydrouracil, cytosine, dihydrocytosine and halogenated uracils.

4. The method of claim 1 wherein said pyrimidine analogue is uracil.

5. The method of claim 1 wherein said silyl blocking member is a member selected from triisopropylsilyl, *tert*-butyltetramethylenesilyl and *tert*-butyldimethylsilyl.

6. The method of claim 1 wherein said moiety is 4-methylumbelliferonyl.

7. The method of claim 1 wherein said moiety is 3-flavonyl.

8. The method of claim 1 wherein said 2',5'-diblocked mononucleoside 3'-phosphodiester is formed by the reaction of said 2',5'-diblocked mononucleoside and a phosphorylated derivative of said moiety.

9. The method of claim 8 wherein said reaction is carried out in the presence of a condensation reagent selected from toluenesulfonyl chloride, mesitylenesulfonyl imidazolide, p-toluenesulfonyl chloride, p-toluenesulfonyl imidazolide, 2,4,6-triisopropylbenzenesulfonyl chloride, mesitylenesulfonyl chloride, picrylsulfonyl chloride, N,N'-dicyclohexylcarbodiimide, and 1-(3-dimethylaminopropyl-)-3-ethylcarbodiimide hydrochloride.

10. The method of claim 9 wherein said reaction is carried out in the presence of an additive selected from N-hydroxysuccinimide, N-hydroxyphthalimide, 2,4,6-triisopropylbenzenesulfonyl chloride and N,N'-dicyclohexylcarbodiimide.

11. The method of claims 8 and 9 wherein the molar ratio of said mononucleoside and said phosphorylated moiety is at least 1 to 1.

12. The method of claim 10 wherein said reaction is carried out in an aprotic polar solvent selected from N,N-dimethylformamide, dioxane and tetrahydrofuran.

13. The method of claim 11 wherein said reaction is carried out in the presence of a base selected from the group consisting of pyridine and triethylamine.

14. The method of claim 13 wherein said base is pyridine.

15. The method of claim 14 wherein said reaction is carried out at a temperature in the range of from −20°C to 25°C.

16. The method of claim 15 wherein said reaction is carried out for a period of from 5 to 18 hours.

17. The method of claim 1 wherein said moiety is an alcohol, the alcohol being phosphorylated *in situ* to form a reactive intermediate, and said reactive intermediate being reacted with said 2',5'-diblocked mononucleoside to form said phosphodiester.

18. The method of claim 1 wherein said 2',5'-diblocked mononucleoside is phosphorylated to form a 2',5'-diblocked mononucleoside reactive intermediate, and reacting said mononucleoside reactive intermediate with said moiety to form said phosphodiester.

19. The method of claim 18 wherein said phosphorylation reaction is carried out in the presence of a phosphorylation reagent selected from phosphorous oxychloride and 2,2,2-trichloroethyl phosphorodichloridite.

20. The method of claim 19 wherein said phosphorylation is carried out in the presence of an aprotic solvent selected from the group consisting of N,N-dimethylformamide, dioxane and tetrahydofuran.

21. The method of claim 20 wherein said phosphorylation is carried out in the presence of a base selected from the group consisting of pyridine and triethylamine.

22. The method of claim 21 wherein said phosphorylation reaction is carried out in the presence of pyridine.

23. The method of claim 22 wherein said phosphorylation reaction is carried out at a temperature in the range of from −10°C to 30°C.

24. The method of claim 23 wherein said phosphorylation reaction is carried out for a time of from 1 hour to 3 hours.

25. The method of claim 18 wherein said moiety is an alcohol.

26. A method for preparing a mononucleoside-3'-phosphodiester-based substrate comprising

a) blocking both the 2'- and 5'-hydroxy groups of a mononucleotide of the formula:

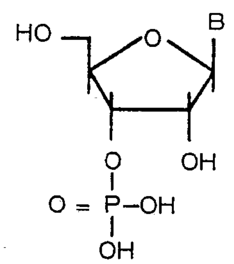

wherein B is a pyrimidine analogue, and wherein the $CH_2OH$ group at the 4'-position is either *cis* or *trans* to the base and the hydroxyl groups at the 2' and 3'-positions are *trans* to the base, by reacting said mononucleotide with a silyl reagent blocking member to form a 2'-, 5'-diblocked mononucleotide of the formula:

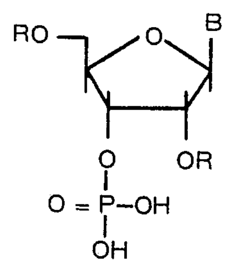

wherein R is a silyl blocking member; and

(b) reacting said 2',5'-diblocked mononucleotide with a chromophore or fluorophore selected from umbelliferonyl, 4-methylumbelliferonyl, 3-flavonyl, o-nitrophenyl, m-nitrophenyl, p-nitrophenyl, 2,4-dinitrophenyl, cyanophenyl, acylphenyl, carboxyphenyl, phenylsulfonate, phenylsulfonyl and phenylsulfoxide, to form a 2',5'-diblocked mononucleoside 3'-phosphodiester of the formula:

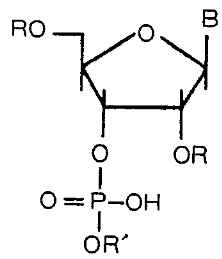

wherein R' is selected from the above group of chromophores and fluorophores.

27. The method of claim 26 wherein as a further step the silyl blocking member at least at the 2'-hydroxyl is removed.

28. The method of claim 27 wherein said removal of said silyl blocking member is carried out in the presence of deblocking reagent selected from *tetra*-butylammonium fluoride, trityltetrafluoroborate, lithium tetra-fluoroborate, hydrogen fluoride, acetic acid and hydrochloric acid.

29. The method of claim 28 wherein said removal of said silyl blocking member is carried out in the solvent selected from tetrahydrofuran, acetonitrile, dioxane and water.

30. The method of claim 29 wherein said deblocking reagent is tetrabutylammonium fluoride, said solvent is tetrahydrofuran and the concentration of tetrabutylammonium fluoride is 1M in said tetrahydrofuran.

31. The method of claim 30 wherein said reaction is carried out at a temperature of from 15°C to 30°C.

32. The method of claim 30 wherein said reaction is carried out for a period of from 20 minutes to 50 minutes.

33. Use of the mononucleoside-3'-phosphodiester-based substrate obtained according to any of the preceding claims for the *in vitro* kinetic monitoring of Ribonuclease A activity.

**Patentansprüche**

1. Verfahren zur Herstellung eines auf einem Mononucleosid-3'-phosphodiester basierenden Substrats, dadurch gekennzeichnet, daß man

(a) sowohl die 2'- als auch die 5'-Hydroxylgruppen eines Mononucleosids der folgenden Formel blockiert

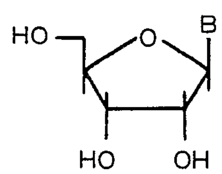

in der B ein Pyrimidin-Analoges ist und in dem die $CH_2OH$-Gruppe in der 4'-Stellung sich entweder in cis- oder trans-Stellung zu der Base befindet und die Hydroxylgruppen in den 2'- und 3'-Stellungen sich in trans-Stellung zur Base befinden, indem man das Mononucleosid mit einem als Blockierungsmittel wirkenden Silylierungsmittel umsetzt, so daß man ein in 2'- oder 5'-Stellung zweifach blockiertes Mononucleosid der folgenden Formel erhält

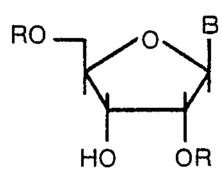

in der R ein Silyl-Blockierungsmittel ist; und

(b) einen in 2'- und 5'-Stellung zweifach blockierten Mononucleosid 3'-phosphodiester der folgenden Formel bildet

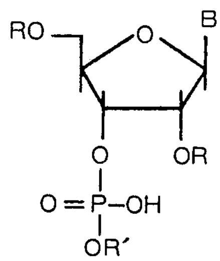

in der R' ein Chromophor oder Fluorophorrest ist, nämlich ein Umbelliferonyl-, 4-Methylumbelliferonyl-, 3-Flavonyl-, o-Nitrophenyl-, m-Nitrophenyl-, p-Nitrophenyl-, 2,4-Dinitrophenyl-, Cyanophenyl-, Acylphenyl-, Carboxyphenyl-, Phenylsulfonat-, Phenylsulfonyl- oder Phenylsulfoxidrest, mit Hilfe eines Verfahrens, bei dem man entweder

1) das in 2'- und 5'-Stellung zweifach blockierte Mononucleosid mit einem Phosphat-Derivat einer der vorstehend genannten Chromophor- oder Fluorophorreste in Gegenwart eines Kondensierungsmittels umsetzt, wobei man den gewünschten Phosphodiester erhält, oder

2) das in 2'- und 5'-Stellung zweifach blockierte Mononucleosid phosphoryliert und das phosphorylierte in 2'- und 5'-Stellung zweifach blockierte Mononucleosid mit einem der vorstehend genannten Chromophore oder Fluorophore in der Alkohol-Form umsetzt, so daß man den gewünschten Phosphodiester erhält.

2. Verfahren nach Anspruch 1, dadurch gekennzeichnet, daß man als weiteren Schritt den in 2'- und 5'-Stellung zweifach blockierten Mononucleosid-3'-phosphodiester mit einem Deblockierungsmittel umsetzt, um das Silyl-Blockierungsmittel zumindest in der 2'-Hydroxyl-Stellung zu entfernen.

3. Verfahren nach Anspruch 1, dadurch gekennzeichnet, daß das Pyrimidin-Analogue ein Uracil, Dihydrouracil, Cytosin, Dihydrocytosin oder ein halogeniertes Uracil ist.

4. Verfahren nach Anspruch 1, dadurch gekennzeichnet, daß das Pyrimidin-Analoge Uracil ist.

5. Verfahren nach Anspruch 1, dadurch gekennzeichnet, daß das Silyl-Blockierungsmittel eine Triisopropylsilyl-, tert.-Butyltetramethylensilyl- oder eine tert.-Butyldimethylsilylgruppe ist.

6. Verfahren nach Anspruch 1, dadurch gekennzeichnet, daß der Rest eine 4-Methylumbelliferonylgruppe ist.

7. Verfahren nach Anspruch 1, dadurch gekennzeichnet, daß der Rest eine 3-Flavonylgruppe ist.

8. Verfahren nach Anspruch 1, dadurch gekennzeichnet, daß der in 2'- und 5'-Stellung zweifach blockierte Mononucleosid-3'-phosphodiester durch die Umsetzung des in 2'- und 5'-Stellung zweifach blockierten Mononucleosids und eines phosphorylierten Derivats des Restes hergestellt wird.

9. Verfahren nach Anspruch 8, dadurch gekennzeichnet, daß die Umsetzung in Gegenwart eines Kondensierungsmittels durchgeführt wird, nämlich Toluolsulfonylchlorid, Mesitylensulfonylimidazolid, p-Toluolsulfonylchlorid, p-Toluolsulfonylimidazolid, 2,4,6-Triisopropylbenzolsulfonylchlorid, Mesitylensulfonylchlorid, Picrylsulfonylchlorid, N,N'-Dicyclohexylcarbodiimid oder 1-(3-Dimethylaminopropyl)-3-äthylcarbodiimidhydrochlorid.

10. Verfahren nach Anspruch 9, dadurch gekennzeichnet, daß die Umsetzung in Gegenwart eines Zusatzes durchgeführt wird, nämlich N-Hydroxysuccinimid, N-Hydroxyphthalimid, 2,4,6-Triisopropylbenzolsulfonylchlorid oder N,N'-Dicyclohexylcarbodiimid.

11. Verfahren nach dem Ansprüchen 8 und 9, dadurch gekennzeichnet, daß das molare Verhältnis des Mononucleosids und des phosphorylierten Restes Mindestens 1:1 beträgt.

12. Verfahren nach Anspruch 10, dadurch gekennzeichnet, daß die Reaktion in einem aprotischen polaren Lösungsmittel durchgeführt wird, nämlich N,N-Dimethylformamid, Dioxan oder Tetrahydrofuran.

13. Verfahren nach Anspruch 11, dadurch gekennzeichnet, daß die Umsetzung in Gegenwart einer Base durchgeführt wird, nämlich Pyridin oder Triäthylamin.

14. Verfahren nach Anspruch 13, dadurch gekennzeichnet, daß die Base Pyridin ist.

15. Verfahren nach Anspruch 14, dadurch gekennzeichnet, daß die Umsetzung bei einer Temperatur im Bereich von −20°C bis 25°C durchgeführt wird.

16. Verfahren nach Anspruch 15, dadurch gekennzeichnet, daß die Umsetzung in einem Zeitraum von 5 bis 18 Stunden durchgeführt wird.

17. Verfahren nach Anspruch 1, dadurch gekennzeichnet, daß der Rest ein Alkohol ist, wobei der Alkohol in situ phosphoryliert wird, so daß eine reaktives Zwischenprodukt entsteht, und das reaktive Zwischenprodukt mit dem in 2'- und 5'-Stellung zweifach blockierten Mononucleosid umgesetzt wird, so daß der Phosphodiester entsteht.

18. Verfahren nach Anspruch 1, dadurch gekennzeichnet, daß das in 2'- und 5'-Stellung zweifach blockierte Mononucleosid phosphoryliert wird, so daß als reaktives Zwischenprodukt ein in 2'- und 5'-Stellung zweifach blockiertes Mononucleosid entsteht, und daß man das als reaktives Zwischenprodukt wirkende Mononucleosid mit dem Rest umsetzt, so daß der Phosphodiester entsteht.

19. Verfahren nach Anspruch 18, dadurch gekennzeichnet, daß die Phosphoryleirungsreaktion in Gegenwart eines Phosphorylierungsmittels durchgeführt wird, nämlich Phosphoroxychlorid oder 2,2,2-Trichloräthylphosphordichloridit.

20. Verfahren nach Anspruch 19, dadurch gekennzeichnet, daß die Phosphorylierung in Gegenwart eines aprotischen Lösungsmittels durchgeführt wird, nämlich N,N-Dimethylformamid, Dioxan oder Tetrahydrofuran.

21. Verfahren nach Anspruch 20, dadurch gekennzeichnet, daß die Phosphorylierungsreaktion in Gegenwart einer Base durchgeführt wird, nämlich Pyridin oder Triäthylamin.

22. Verfahren nach Anspruch 21, dadurch gekennzeichnet, daß die Phosphorylierungsreaktion in Gegenwart von Pyridin durchgeführt wird.

23. Verfahren nach Anspruch 22, dadurch gekennzeichnet, daß die Phosphorylierungsreaktion bei einer Temperatur im Bereich von −10°C bis 30°C durchgeführt wird.

24. Verfahren nach Anspruch 23, dadurch gekennzeichnet, daß die Phosphorylierungsreaktion in einem Zeitraum von 1 bis 3 Stunden durchgeführt wird.

25. Verfahren nach Anspruch 18, dadurch gekennzeichnet, daß der rest ein Alkohol ist.

26. Verfahren zur Herstellung eines auf einem Mononucleosid-3'-phosphodiester basierenden Substrats, dadurch gekennzeichnet, daß man

a) sowohl die 2'- als auch die 5'-Hydroxylgruppen eines Mononucleotids der folgenden Formel blockiert

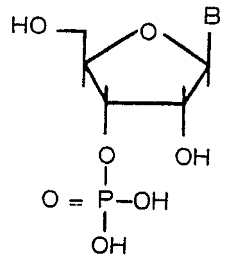

in der B ein Pyrimidin-Analoges bedeutet, und in dem die $CH_2OH$-Gruppe in der 4'-Stellung sich entweder in cis- oder trans-Stellung zur Base befindet und die Hydroxylgruppen in den 2'- und 3'-Stellungen sich in trans-Stellung zur Base finden, indem man das Mononucleotid mit einem als Blockierungsmittel wirkenden Silylierungsmittel umsetzt, so daß ein in 2'- und 5'-Stellung zweifach blockiertes Mononucleotid der folgenden Formel entsteht

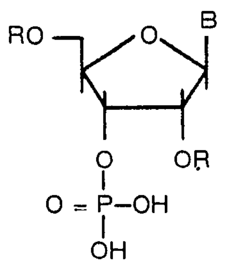

in der R ein Silylblockierungsmittel ist; und

b) das in 2'- und 5'-Stellung zweifach blockierte Mononucleotid mit einem Chromophor oder einem Fluorophor umsetzt, nämlich einer Umbelliferonyl-, 4-Methylumbelliferonyl-, 3-Flavonyl-, o-Nitrophenyl-, m-Nitrophenyl-, p-Nitrophenyl-, 2,4-Dinitrophenyl-, Cyanophenyl-, Acylphenyl-, Carboxyphenyl-, Phenylsulfonat-, Phenylsulfonyl- oder Phenylsulfoxidgruppe, so daß man einen in 2'- und 5'-Stellung zweifach blockierten Mononucleosid-3'-phosphodiester der folgenden Formel erhält

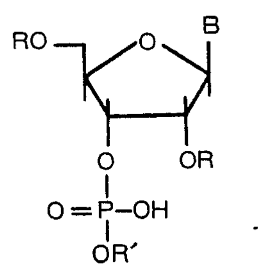

in der R' einen der vorstehend beschriebenen Chromophor- oder Fluorphorreste bedeutet.

27. Verfahren nach Anspruch 26, dadurch gekennzeichnet, daß man als weiteren Schritt das Silylblockierungsmittel zumindest in der 2'-Hydroxyl-Stellung entfernt.

28. Verfahren nach Anspruch 27, dadurch gekennzeichnet, daß die Entfernung des Silylblockierungsmittels in Gegenwart eines Deblockierungsmittels durchgeführt wird, nämlich Tetrabutylammoniumfluorid, Trityltetrafluoroborat, Lithiumtetrafluoroborat, Fluorwasserstoff, Essigsäure oder Chlorwasserstoffsäure.

29. Verfahren nach Anspruch 28, dadurch gekennzeichnet, daß die Entfernung des Silylblockierungsmittels in einem Lösungsmittel durchgeführt wird, nämlich in Tetrahydrofuran, Acetonitril, Dioxan oder Wasser.

30. Verfahren nach Anspruch 29, dadurch gekennzeichnet, daß das Deblockierungsmittel Tetrabutylammoniumfluorid ist, das Lösungsmittel Tetrahydrofuran ist und die Konzentration von Tetrabutylammoniumfluorid 1M in Tetrahydrofuran ist.

31. Verfahren nach Anspruch 30, dadurch gekennzeichnet, daß die Umsetzung bei einer Temperatur von 15°C bis 30°C durchgeführt wird.

32. Verfahren nach Anspruch 30, dadurch gekennzeichnet, daß die Umsetzung in einem Zeitraum von 20 bis 50 Minuten durchgeführt wird.

33. Verwendung des auf einem Mononucleosid-3'-phosphodiester basierenden Substrats, das gemäß einem der vorangehenden Ansprüche erhalten wurde, für die in vitro-kinetische Bestimmung von Ribonuclease A-Aktivität.

**Revendications**

1. Procédé de préparation d'un substrat à base de mononucléoside-3'-phosphodiester comprenant:
(a) le blocage des groupes hydroxyle en 2' et 5' d'un mononucléoside de la formule:

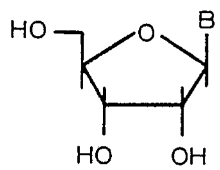

dans laquelle B représente un analogue de pyrimidine, et dans laquelle le groupe $CH_2OH$ en position 4' est soit en *cis* soit en *trans* par rapport à la base et les groupes hydroxyle en positions 2' et 3' sont en *trans* par rapport à la base, en faisant réagir le mononucléoside avec un élément de blocage réactif de silylation pour former un mononucléoside dibloqué en 2', 5' de la formule:

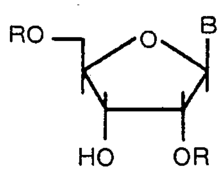

dans laquelle R représente un élément de blocage silylé; et
(b) la formation d'un mononucléoside-3'-phosphodiester dibloqué en 2', 5' de la formule:

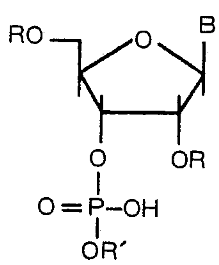

dans laquelle R' représente un fragment de chromophore ou de fluorophore choisi parmi l'umbelliferonyle, le 4-méthylumbelliferonyle, le 3-flavonyle, l'o-nitrophényle, le m-nitrophényle, le p-nitrophényle, le 2,4-dinitrophényle, le cyanophényle, l'acylphényle, le carboxyphényle, le phénylsulfonate, le phénylsulfonyle et le phénylsulfoxyde, par un procédé choisi parmi:

1) la mise en réaction du mononucléoside dibloqué en 2', 5' avec un dérivé phosphaté de l'un des fragments de chromophore ou de fluorophore précités en présence d'un agent de condensation pour former le phosphodiester désiré, ou

2) la phosphorylation du mononucléoside dibloqué en 2', 5' et la mise en réaction du mononucléoside dibloqué en 2', 5' phosphorylé avec l'un des chromophores ou fluorophores précités sous forme alcoolique pour former le phosphodiester désiré.

2. Procédé suivant la revendication 1, caractérisé en ce qu'il comprend en outre la mise en réaction du

mononucléoside-3'-phosphodiester dibloqué en 2', 5' avec un agent de déblocage pour séparer l'élément de blocage silylé au moins en l'hydroxyle 2'.

3. Procédé suivant la revendication 1, caractérisé en ce que l'analogue de pyrimidine est un élément choisi parmi l'uracile, le dihydrouracile, la cytosine, la dihydrocytosine et les uraciles halogénés.

4. Procédé suivant la revendication 1, caractérisé en ce que l'analogue de pyrimidine est l'uracile.

5. Procédé suivant la revendication 1, caractérisé en ce que l'élément de blocage silylé est un membre choisi parmi le triisopropylsilyle, le *tert*-butyltétraméthylènesilyle et le *tert*-butyldiméthylsilyle.

6. Procédé suivant la revendication 1, caractérisé en ce que le fragment est le 4-méthylumbelliferonyle.

7. Procédé suivant la revendication 1, caractérisé en ce que le fragment est le 3-flavonyle.

8. Procédé suivant la revendication 1, caractérisé en ce que le mononucléoside-3'-phosphodiester dibloqué en 2', 5' est formé par la réaction du mononucléoside dibloqué en 2', 5' et d'un dérivé phosphorylé du fragment précité.

9. Procédé suivant la revendication 8, caractérisé en ce que la réaction est réalisée en pésence d'un réactif de condensation choisi parmi le chlorure de toluènesulfonyle, l'imidazolide de mésitylènesulfonyle, le chlorure de p-toluènesulfonyle, l'imidazolide de p-toluènesulfonyle, le chlorure de 2,4,6-triisopropylbenzènesulfonyle, le chlorure de mésitylènesulfonyle, le chlorure de picrylsulfonyle, le N,N'-dicyclohexylcarbodiimide et le chlorhydrate de 1-(3-diméthylaminopropyl)-3-éthylcarbodiimide.

10. Procédé suivant la revendication 9, caractérisé en ce que l'on réalise la réaction en présence d'un additif choisi parmi le N-hydroxysuccinimide, le N-hydroxyphtalimide, le chlorure de 2,4,6,-triisopropylbenzènesulfonyle et le N,N'-dicyclohexylcarbodiimide.

11. Procédé suivant l'une ou l'autre des revendications 8 et 9, caractérisé en ce que le rapport molaire du mononucléoside et du fragment phosphorylé est d'au moins 1/1.

12. Procédé suivant la revendication 10, caractérisé en ce que l'on réalise la réaction dans un solvant polaire neutre choisi parmi le N,N-diméthylformamide, le dioxanne et le tétrahydrofuranne.

13. Procédé suivant la revendication 11, caractérisé en ce que l'on réalise la réaction d'une base choisie dans le groupe comprenant la pyridine et la triéthylamine.

14. Procédé suivant la revendication 13, caractérisé en ce que la base est la pyridine.

15. Procédé suivant la revendication 14, caractérisé en ce que l'on réalise la réaction à une température de l'ordre de −20°C à 25°C.

16. Procédé suivant la revendication 15, caractérisé en ce que l'on réalise la réaction pendant une période de 5 à 18 heures.

17. Procédé suivant la revendication 1, caractérisé en ce que le fragment précité est un alcool, l'alcool étant phosphorylé *in situ* pour former un produit intermédiaire réactif, et le produit intermédiaire réactif étant mis en réaction avec le mononucléoside dibloqué en 2', 5' pour former le phosphodiester.

18. Procédé suivant la revendication 1, caractérisé en ce que le mononucléoside dibloqué en 2', 5' est phosphorylé pour former un intermédiaire réactif de mononucléoside dibloqué en 2', 5', cet intermédiaire réactif de mononucléoside étant mis en réaction avec le fragment précité pour former le phosphodiester.

19. Procédé suivant la revendication 18, caractérisé en ce que l'on réalise la réaction de phosphorylation en présence d'un réactif de phosphorylation choisi parmi l'oxychlorure phosphoreux et la 2,2,2-trichloroéthyl phosphorodichloridite.

20. Procédé suivant la revendication 19, caractérisé en ce que l'on réalise la phosphorylation en présence d'un solvant neutre choisi dans le groupe comprenant le N,N-diméthylformamide le dioxanne et de tétrahydrofuranne.

21. Procédé suivant la revendication 20, caractérisé en ce que l'on réalise la réaction de phosphorylation en présence d'une base choisie dans le groupe comprenant la pyridine et la triéthylamine.

22. Procédé suivant la revendication 21, caractérisé en ce qu'on réalise la réaction de phosphorylation en présence de pyridine.

23. Procédé suivant la revendication 22, caractérisé en ce qu'on réalise la réaction de phosphorylation à une température de l'ordre de −10°C à 30°C.

24. Procédé suivant la revendication 23, caractérisé en ce que l'on réalise la réaction de phosphorylation pendant une période d'lheure à 3 heures.

25. Procédé suivant la revendication 18, caractérisé en ce que le fragment précité est un alcool.

26. Procédé de préparation d'un substrat à base de mononucléoside-3'-phosphodiester, comprenant:

a) le blocage des groupes hydroxyle en 2' et 5' d'un mononucléotide de la formule:

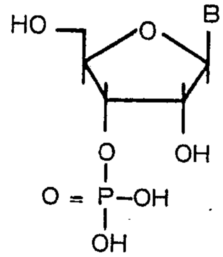

dans laquelle B est un analogue de pyrimide, et dans laquelle le groupe $CH_2OH$ en position 4′ est en *cis* ou en *trans* par rapport à la base et les groupes hydroxyle en positions 2′ et 3′ sont en *trans* par rapport à la base, en faisant réagir le mononucléotide avec un élément de blocage réactif silylé pour former un mononucléotide dibloqué en 2′, 5′ de la formule:

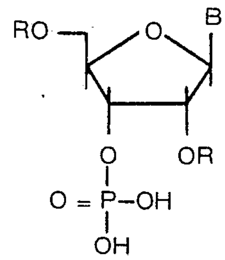

dans laquelle R représente en élément de blocage silylé; et

b) la mise en réaction de ce mononucléotide dibloqué en 2′, 5′ avec un chromophore ou fluorophore choisi parmi l'umbelliferonyle, le 4-méthylumbelliferonyle, le 3-flavonyle, l'o-nitrophényle, le m-nitrophényle, le p-nitrophényle, le 2,4-dinitrophényle, le cyanophényle, l'acylphényle, le carboxyphényle, le phénylsulfonate, le phénylsulfonyle et le phénylsulfoxyde, pour former un mononucléoside-3′-phosphodiester dibloqué en 2′, 5′ de la formule:

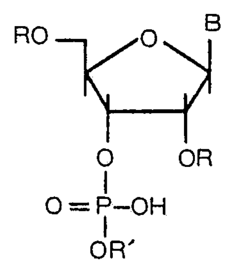

dans laquelle R′ est choisi dans le groupe précité de chromophores et de fluorophores.

27. Procédé suivant la revendication 26, caractérisé en ce que, comme étape ultérieure, on sépare l'élément de blocage silylé au moins en l'hydroxyle 2′.

28. Procédé suivant la revendication 27, caractérisé en ce que la séparation de l'élément de blocage silylé est réalisée en présence d'un réactif de déblocage choisi parmi le fluorure de tétrabutylammonium, le trityltétrafluoroborate, le tétrafluoroborate de lithium, l'acide fluorhydrique, l'acide acétique et l'acide chlorhydrique.

29. Procédé suivant la revendication 28, caractérisé en ce que la séparation de l'élément de blocage silylé est réalisée dans un solvant choisi parmi le tétrahydrofuranne, l'acétonitrile, le dioxanne et l'eau.

30. Procédé suivant la revendication 29, caractérisé en ce que le réactif de déblocage est le fluorure de tétrabutylammonium, le solvant est le tétrahydrofuranne et la concentration en fluorure de tétrabutylammonium est de 1M dans le tétrahydrofuranne.

31. Procédé suivant la revendication 30, caractérisé en ce que l'on réalise la réaction à une température de 15°C à 30°C.

32. Procédé suivant la revendication 30, caractérisé en ce que l'on réalise la réaction pendant une période de 20 minutes à 50 minutes.

33. Utilisation du substrat à base de mononucléoside-3′-phosphodiester obtenu suivant l'une quelconque des revendications précédentes, pour le contrôle cinétique in vitro de l'activité de ribonucléase A.